Europäisches Patentamt

European Patent Office

Office européen des brevets

(19)

(11) Veröffentlichungsnummer: **0 450 111 A1**

## (12) EUROPÄISCHE PATENTANMELDUNG

(21) Anmeldenummer: 90106250.5

(22) Anmeldetag: 31.03.90

(51) Int. Cl.5: **A61B 1/06**, A61B 17/36,
A61B 1/00, A61B 1/12,
A61B 17/34

(43) Veröffentlichungstag der Anmeldung:
09.10.91 Patentblatt 91/41

(84) Benannte Vertragsstaaten:
**DE FR GB IT SE**

(71) Anmelder: **Auer, Ludwig, Univ. Prof. Dr.
Neurochir. Klinik Nordstadt Krankenhaus
W-3000 Hannover 1(DE)**

(72) Erfinder: **Auer, Ludwig, Univ. Prof. Dr.
Neurochir. Klinik Nordstadt Krankenhaus
W-3000 Hannover 1(DE)**

(74) Vertreter: **Gödl, Oswald, Dipl.-Ing. (FH)
Schnideritschstrasse 12
A-8045 Graz(AT)**

(54) Verwendung der Computertomographie, Magnetresonanztomographie, Ultraschallbildgebung od. dgl. bildgebende Verfahren zur Leitung für endoskopische Diagnostik und operative Therapie.

(57) Die Erfindung betrifft die Verwendung der Computertomographie, Magnetresonanztomographie, Ultraschallbildgebung od. dgl. bildgebende Verfahren zur Leitung für endoskopische Diagnostik und operative Therapie zur Behandlung krankhafter Veränderungen in der Schädelhöhle. Hierbei werden diese Aufzeichnungsträger sowohl für eine visuelle Kontrolle als auch die erfaßten Daten für eine äußerst präzise Positionierung im Zielgebiet herangezogen.

EP 0 450 111 A1

Die Erfindung betrifft die Verwendung der Computertomographie, Magnetresonanztomographie, Ultraschallbildgebung od. dgl. bildgebende Verfahren zur Leitung für endoskopische Diagnostik und operative Therapie zur Behandlung krankhafter Veränderungen in der Schädelhöhle entlang eines durch Trepanation geschaffenen Zugangsweges als Aufzeichnungsträger zur visuellen Kontrolle und Datenerfassung.

Diese bildgebenden Verfahren im Zusammenhang mit der endoskopischen Diagnostik und operativen Therapie dienen dazu, krankhafte Stellen innerhalb der Schädelhöhle, z.B. des Gehirns oder des Hirnkammersystems, auf einen miniaturisierten Zugangsweg gezielt zu erreichen und in diesem Zielgebiet operative Schritte unternehmen zu können und darüberhinaus die Positionierung im Zielgebiet wiederholbar durchführen zu können.

Es ist das Einbringen von Sonden in Zielgebiete innerhalb des Gehirns bekannt, wobei die Methode der Stereotaxie dem Fachmann ein vertrauter Begriff ist.

Die herkömmliche Operationsmethode der Kraniotomie hat den Nachteil, daß ein Zugangsweg erforderlich ist. So ist es durchaus üblich, einen großen zylindrischen und vorallem gesunden Hirngewebsteil mit einem Mindestdurchmesser von 2 bis 3 cm und mehr zu beseitigen, um überhaupt an das Zielgebiet zu gelangen und operative Maßnahmen zu ergreifen. Für medizinische Untersuchungen und operative Eingriffe sind als Endoskope bezeichnete Einrichtungen bekannt. Diese sind rohrförmige Geräte, die sich zum Einführen in Körperhöhlen eignen und in ihrem Aufbau sehr ähnlich sind. Es wird demnach angestrebt, diese Endoskope möglichst dünn und schlank auszuführen, um die natürlichen Zugangswege nicht vergrößern zu müssen. Im Inneren befindet sich meist eine mehrlinsige Optik, die etwas vergrößert. Das vordere Ende trägt eine Kaltlichtfaser. Bei manchen Konstruktionen ist vorn noch ein Spiegel oder ein Prisma zur Umlenkung des Beobachtungsganges und zur seitwärtigen Betrachtung. Meist sind noch Spülkanäle untergebracht, weil zur Betrachtung der betreffenden Körperhöhle deren Aufblähung oder Klarspülung notwendig ist.

Die Operationsendoskope sind grundsätzlich ebenso aufgebaut, haben jedoch einen Kanal zum Durchschieben von Zangen und Geweben. Auch elektrische Schwachstromglühbrenner zur Kaustik und Elektroden zur Gewebekoagulation sind üblich. In der Regel sind Endoskope besonders für ihren speziellen Verwendungszweck und Einsatzbereich unterschiedlich ausgebildet.

Es ist jedoch nicht damit getan, spezielle Endoskope einzusetzen, sondern es ist auch die Forderung gestellt, die gewünschte Stelle, d.h. im Zielgebiet sehr präzise zu erreichen. Dies erfordert jedoch einen umfangreichen apparativen Aufbau sowie komplizierte Rechenvorgänge.

Nach DE-OS 34 43 337 ist ein Instrument zur Untersuchung und Behandlung von Verengungen in Körperhöhlen oder Gefäßen bekannt, um durch visuelle Untersuchung diese festzustellen und diese auch unter direkter visueller Kontrolle zu beheben. Hierbei wird ein vollwandiger Schaft als Katheter verwendet, der über seinen Querschnitt verteilte und über seine Länge verlaufende Kanäle gleichen oder ungleichen Querschnitts zum Zu- und Abführen von Spülflüssigkeiten, Körpersekreten od. dgl., aufweist und zum Durchführen von Hilfsinstrumenten und Meßelementen sowie zur Aufnahme von Fiberlicht- und Fiberbildleitern dient.

Weiters ist nach DE-PS 30 40 335 eine flexible Rohranordnung für ein Endoskop bekannt, das ebenfalls einen vollwandigen Schaft aufweist, jedoch die Kanäle für die Aufnahme und Führung der zur Endoskopie notwendigen Vorrichtungen als flexible Rohre in der Oberfläche des Schaftes in Nuten liegend, gebildet sind. Hierbei wird angestrebt, bei relativ hoher Flexibilität der Rohranordnung bestimmte flexible Rohre in einfacher Weise auszutauschen.

Diesen flexiblen Rohranordnungen bzw. vollwandigen Schäften ist jedoch der Nachteil gemeinsam, daß eine wiederholbare, genaue Positionierung in der Schädelhöhle nicht möglich ist, weil ein Verbiegen beim geringsten Hindernis gegeben ist.

Ferner ist nach DE-PS 29 51 765 ein flexibles Endoskop zur Untersuchung von Körperhöhlen bekannt, das ein optisches Betrachtungssystem aufweist, mit dessen Hilfe das Endstück so überwacht werden kann, daß es im Zentrum des Darmquerschnittes gerichtet bleibt. Hierbei bedient man sich einer Mehrzahl von Ultraschallwandlern als Sender und Empfänger, die im Abstand voneinander angeordnet sind, aus deren Signale die Abstände des distalen Endstückes zu den Wänden der Körperhöhle bestimmt werden.

Schließlich ist nach DE-AS 29 24 494 eine Vorrichtung bekannt, die zur Verhinderung des Beschlagens des Beobachtungsfensters eines Endoskopes durch Beheizen dient. Durch Infrarotstrahlen, d.h. Wärmestrahlen, die zusätzlich zu den sichtbaren Lichtstrahlen durch optische Lichtleiterbündel geführt werden, wird das Beobachtungsfenster aufgeheizt.

Der Erfindung liegt die Aufgabe zugrunde, durch Aufzeichnungsträger den Zugang im Zielgebiet sehr präzise zu erreichen und sowohl eine visuelle Kontrolle als auch eine wiederholbare Positionierung zu ermöglichen. Die Lösung dieser Aufgabe besteht erfindungsgemäß aus den Angaben des Hauptanspruches.

Durch diese Ausbildung wird erreicht, daß das Führungsrohr des Endoskopes, in dem alle notwendigen Leitungen für einen miniaturisierten Zugangsweg untergebracht sind, präzise und wiederholbar an das Zielgebiet heranführbar ist.

Vorteilhafte Weiterbildungen der Erfindung sind in den Unteransprüchen angegeben.

Anhand eines Ausführungsbeispiels sei die Erfindung näher erläutert. Es zeigen:

Fig. 1    Schematische Darstellung der Einrichtung

Fig. 2    Ausbildung der Rohrspitze

Fig. 3    Querschnitt des Führungsrohres.

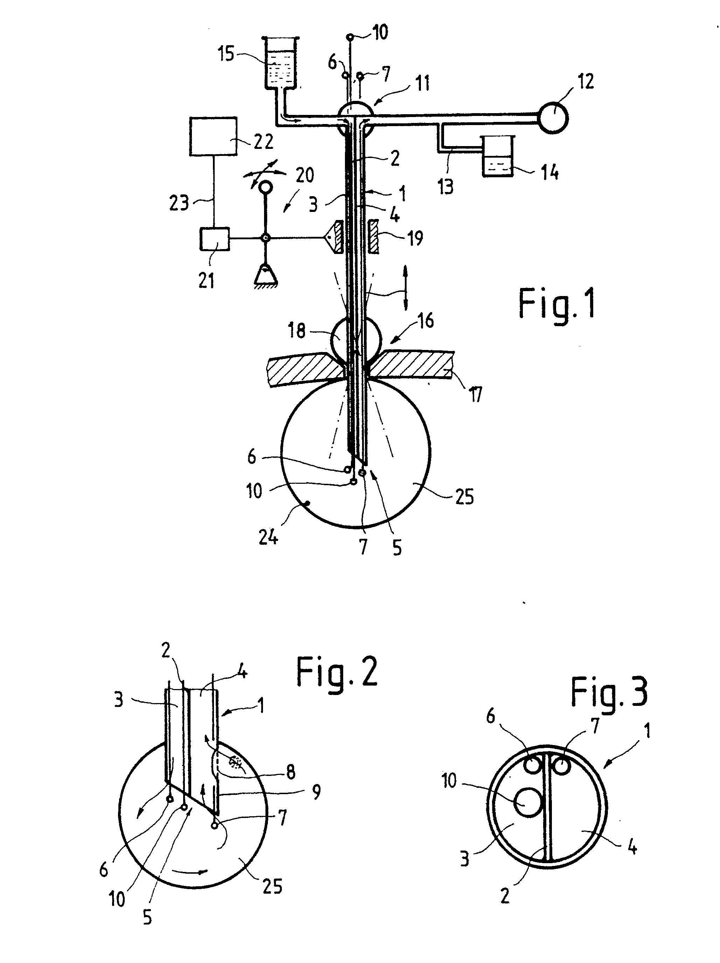

Die Fig. 1 zeigt schematisch die Anordnung der Einzelteile und deren Zuordnung für die endoskopische Operation unter Zuhilfenahme bildgebender Verfahren. Ein Führungsrohr (1) enthält einen Mittelsteg (2), der das Führungsrohr (1) in wenigstens einen Zuführungskanal (3) und einen Rückführungskanal (4) unterteilt. Am Ende des Führungsrohres (1) ist die Rohrspitze (5), die in eine Abschrägung von beispielsweise 30° bis 60°, vorzugsweise 45°, aufweist. Im Inneren des Führungsrohres (1) ist zusätzlich zu dem Zuführungskanal (3) noch ein Instrumentenkanal (6) und ein Kanal (10) für das optische System untergebracht. Im Rückführungskanal (4) hingegen ist die Mikrolasersonde (7) verlegt. Diese zusätzlichen Kanäle können auch schwimmend angeordnet sein. Am oberen Ende des Führungsrohres (1) ist ein Dreiwegehahn (11) angeordnet. Hiermit kann man den Zufluß, der über ein Gefäß (15) erfolgt, steuern. Eine Saugpumpe (12) sorgt für den Abfluß der Spüllösung. An der Abflußleitung vor der Saugpumpe (12) ist noch ein Abführschlauch (13) angeschlossen und mit einem in der Höhe nach verstellbaren Ablaufgefäß (14) verbunden. Dies dient dazu, den Druck der Flüssigkeit an der Rohrspitze (5), d.h. der Spüllösung je nach Höhenstellung des Ablaufgefäßes (14), zu regulieren. Das starre Führungsrohr (1) ist in einer Kugelführung (18) allseitig verschwenkbar und axial verschiebbar gelagert. (Durch Doppelpfeil angedeutet). Damit kann man mit der Rohrspitze (5) jede gewünschte Position, z.B. Punkt 24 in der Schädelhöhle (25) erreichen. Die Kugelführung (18) ist beispielsweise an der Zugangsöffnung (16) der Schädeldecke (17) angeordnet. Die Art der Befestigung ist dem Fachmann bekannt und es braucht darauf nicht näher eingegangen zu werden. Ferner ist noch eine Führung (19) für das Führungsrohr (1) vorgesehen. An dieser Führung (19) ist eine Hebelkombination (20) angeschlossen, die es nun ermöglicht, durch allseitige Verstellbewegung - wie durch Kreuzpfeil angegeben - das Führungsrohr (1) zu positionieren. An diese Hebelkombination (20) ist eine Motorik (21) angegliedert, mit der man die Verstellbewegung durch einen Servomotor durchführen kann. Durch eine entsprechende Übersetzung ist eine exakte Verstellbarkeit und Feineinstellung garantiert. Über Leitungen (23) ist ein Aufzeichnungsträger (22) angeschlossen, wobei zur Datenerfassung die Computertomographie, Magnetresonanztomographie, Ultraschallbildgebung od. dgl. bildgebende Verfahren verwendet werden. Sowohl für die visuelle Kontrolle als auch die erfaßten Daten sind für eine äußerst exakte Positionierung für die endoskopische Diagnostik und operative Therapie von großer Wichtigkeit. Dieser Aufzeichnungsträger (22) steht mit dem Instrumentenkanal (10) für das optische System in Verbindung und damit kann man einen direkten Sichtkontakt zu den räumlichen Gegebenheiten in der Umgebung der Rohrspitze (5) herstellen.

In Fig. 2 ist vergrößert die Rohrspitze (5) des Führungsrohres (1) dargestellt. Es zeigt den Mittelsteg (2), den Zuführungskanal (3) sowie den Rückführungskanal (4). Ferner ist der Instrumentenkanal (6) zu erkennen, der zum Einbringen mikroskopischer Instrumente, wie Schere, Biopsiezange, Punktionsnadel, zusätzlicher Absaugkatheter, monopolare Koagulationssonde, verwendet wird. Die Mikrolasersonde (7) hingegen wird als Schneidegerät für abzutrennende Gewebeteile, aber auch als Kougulationsgerät für blutende Gefäße verwendet. Der Kanal (10) dient wiederum für die Kaltlichtzufuhr od. dgl. Einrichtungen. Wie schon erwähnt, hat das Führungsrohr (1) an seinem Ende eine Abschrägung zur Bildung der Rohrspitze (5). Am längeren Teil der Abschrägung ist noch eine Öffnung (8) vorgesehen, damit der Abtransport der Spüllösung, d.h. der Restspülmenge oder Gewebe od. dgl. nicht gestört ist, oder eine Staubildung verursacht. Der verbleibende Wandteil (9) entspricht in seiner Längenersteckung dem Durchmesser der runden Öffnung (8). Ferner sind noch Strömungspfeile angegeben, woraus zu entnehmen ist, wie der Strömungsverlauf der Spüllösung in der Schädelhöhle (25) erfolgt.

Die Fig. 3 zeigt einen vergrößerten Querschnitt des Führungsrohres (1). Dieser veranschaulicht die Lage des Mittelsteges (2) und die Unterbringung des Instrumentenkanals (6), der Mikrolasersonde (7) sowie des Kanals (10) für das optische System.

Wesentlich ist, daß diese Ausbildung des Führungsrohres (1) eine ultraschallgeleitete endoskopische Diagnostik und operative Therapie ermöglicht und alle notwendigen Kanäle und Leitungen sowie Sonden und dergleichen enthält, ohne daß die Öffnung in der Schädeldecke mehr als einige Millimeter Durchmesser betragen muß. Darüberhinaus ist durch die Verwendung der Computertomographie, Magnetresonanztomographie, Ultraschallbildgebung od. dgl. bildgebende Verahren zur Leitung für endoskopische Diagnostik und operative Therapie eine exakte und wiederholbare Positionierung einwandfrei gegeben.

**Patentansprüche**

1. Verwendung der Computertomographie, Magnetresonanztomographie, Ultraschallbildgebung od. dgl. bildgebende Verfahren für endoskopische Diagnostik und operative Therapie zur Behandlung krankhafter Veränderungen in der Schädelhöhle entlang eines durch Trepanation geschaffenen Zugangsweges, durch welchen mittels Punktion ein starres Führungsrohr (1) vorschiebbar ist, das in Längsrichtung in voneinander unabhängige, durchgehende Kanäle unterteilt, außerhalb des Operationsbereichs verstellbar gelagert und zum Zu- und Abführen von Flüssigkeit unter Druck an seinem offenen distalen Ende ausgebildet ist und zum Durchführen von Hilfsinstrumenten und Meßelementen sowie zur Aufnahme von Licht- und Bildleitern dient, wobei zur Positionierung die bildgebenden Verfahren seiner am distalen Ende ausgebildeten Rohrspitze (5) eine an der Zugangsöffnung (16) der Schädeldecke (17) befestigbare Kugelführung (18) zugeordnet ist, in welcher das Führungsrohr (1) zu seiner allseitigen Bewegungsfreiheit axial verschiebbar und allseitig verschwenkbar gelagert ist, und daß eine weitere Führung (19) für das Führungsrohr (1) vorgesehen ist, welche mit einer Hebelkombination (20) gelenkig verbunden ist, an der eine ultraschallgesteuerte dreidimensionale Motorik (21) anschließbar ist, durch die die Rohrspitze (5) automatisch an einem im Bild vorgewählten Punkt z.B. in der Schädelhöhle (25) führbar ist.

2. Verwendung bildgebender Verfahren nach Anspruch 1, gekennzeichnet durch einen im Rückführungskanal (4) eingebrachte Mikrolasersonde (7), die zur automatischen dreidimensionalen Führung dient und in einem um die Rohrspitze (5) gelegenen, annähernd kugelförmigen Hohlraum elektronisch steuerbar ist.

3. Verwendung bildgebender Verfahren nach Anspruch 1, gekennzeichnet durch einen Zuführungskanal (3) in dem ein optisches System (10), z.B. ein Lichtleiter und ein Instrumentenkanal (6) vorgesehen sind, während im Rückführungskanal (4) eine Mikrolasersonde (7) eingebracht sind.

4. Verwendung bildgebender Verfahren nach Anspruch 1 bis 3, gekennzeichnet durch ein optisches System (10), das an eine Videokamera, die den direkten Sichtkontakt der räumlichen Gegebenheiten in der Umgebung der Rohrspitze (5) erlaubt, und an ein TV-Gerät (22) anschließbar ist.

5. Verwendung bildgebender Verfahren nach einem der Ansprüche 1 bis 4, gekennzeichnet durch einen am proximalen Ende des Führungsrohres (1) angeordneten Dreiwegehahn (11),welcher den Zuführungskanal (3) mit einem Gefäß (15) für die Spüllösung und den Rückführungskanal (4) mit einer Saugpumpe (12) verbindet, und daß von der Leitung zwischen Dreiwegehahn (11) und Saugpumpe (12) ein weiterer Abführschlauch (13) abzweigt, der in ein höhenverstellbares Ablaufgefäß (14) mündet, um den Druck in der Spüllösung im Bereich der Rohrspitze (5) je nach Höhenlage des Ablaufgefäßes regulieren zu können.

6. Verwendung bildgebender Verfahren nach einem der Ansprüche 1 bis 5, gekennzeichnet durch eine im Bereich der Rohrspitze (5) im längeren Wandteil des Führungsrohres (1) in den Rückführungskanal (4) mündende seitliche Öffnung (8), wobei diese Öffnung (8) und der Rückführungskanal (4) den gleichen Querschnitt aufweisen, und daß der verbleibende Wandteil (9) zwischen Rohrspitze (5) und seitlicher Öffnung (8) in seiner Längserstreckung dem Durchmesser der seitlichen Öffnung (8) entspricht, während im Zuführungskanal (3) und Rückführungskanal (4) weitere abgeschlossene Kanäle (6,7) schwimmend untergebracht sind.

7. Verwendung bildgebender Verfahren nach Anspruch 5 oder 6, gekennzeichnet durch eine im Rückführungskanal (4) eingebrachte Mikrolasersonde (7), die sowohl als Schneidegerät für abzutrennende Gewebeteile, als auch als Koagulationsgerät für blutende Gefäße einsetzbar ist.

8. Verwendung bildgebender Verfahren nach Anspruch 5 bis 7, gekennzeichnet durch einen im Zuführungskanal (3) vorgesehenen Instrumentenkanal (6), der zum Einbringen mikroskopischer Operationsinstrumente, wie Schere, Biopsiezange, Punktionsnadel, zusätzlicher Absaugkatheter, monopolare Koagulationssonde, dient.

Fig.1

Fig.2

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-C-3 742 708 (L. AUER)<br>* Spalte 4, Zeile 58 - Spalte 6, Zeile 2; Figuren 1-3 *<br>- - - | 1-8 | A 61 B 1/06<br>A 61 B 17/36<br>A 61 B 1/00<br>A 61 B 1/12<br>A 61 B 17/34 |
| Y | PROCEEDINGS OF THE SEVENTH ANNUAL CONFERENCE OF THE IEEE/ENGINEERING IN MEDICINE AND BIOLOGYBand 2, 27. - 30. September 1985, Seiten 692-695, Chicago, US; B.A. KALL et al.: "Stereostatic Computer-Aided Neurosurgery: CO2 Laser Resection of CNS Tumors"<br>* Seite 693, linke Spalte, Zeile 22 - rechte Spalte, Zeile 20; Figuren 2,3 *<br>- - - | 1 | |
| Y | WO-A-8 701 273 (R.K. FOX et al.)<br>* Zusammenfassung; Seite 15, Zeile 1 - Seite 17, Zeile 24; Seite 21, Zeile 3 - Seite 22, Zeile 25; Figuren 1,5 * | 1 | |
| A | | 2-5,7 | |
| | - - - | | |
| A | EP-A-0 269 791 (MESSERSCHMITT-BOELKOW-BLOHM GMBH)<br>* Spalte 3, Zeile 12 - Spalte 4, Zeile 8; Figuren 1-3 *<br>- - - | 1,4 | |
| A | GB-A-2 017 506 (D.S.J. CHOY)<br>* Seite 2, Zeile 63 - Seite 3, Zeile 47; Figuren 1-4 *<br>- - - - | 1,5 | |

RECHERCHIERTE SACHGEBIETE (Int. Cl.5)

A 61 B 1/00
A 61 B 17/00
A 61 M 25/00

Der vorliegende Recherchenbericht wurde für alle Patentansprüche erstellt

| Recherchenort | Abschlußdatum der Recherche | Prüfer |
|---|---|---|
| Berlin | 26 November 90 | WEIHS J.A. |